# EUROPEAN PATENT SPECIFICATION

(11) **EP 4 072 614 B1**
(45) Date of publication and mention of the grant of the patent: **08.01.2025**
(21) Application number: 20772066.5
(22) Date of filing: 18.09.2020
(51) Int. Cl.: A61M 1/36, A61M 39/00, A61M 5/14, A61M 39/10, A61M 5/142

(54) **FLUID DIVERTING DEVICE FOR AN APPARATUS FOR EXTRACORPOREAL TREATMENT OF BLOOD AND BLOOD SET PROVIDED WITH SAID FLUID DIVERTING DEVICE**
ZUSATZMODUL FÜR EINE VORRICHTUNG ZUR EXTRAKORPORALEN BEHANDLUNG VON BLUT UND MIT BESAGTEM ZUSATZMODUL AUSGESTATTETER BLUTSATZ
MODULE COMPLÉMENTAIRE DE TRAITEMENT EXTRACORPOREL DU SANG ET ENSEMBLE DE SANG FOURNI AVEC LEDIT MODULE COMPLÉMENTAIRE

(30) Priority: 13.12.2019 EP 19216147
(43) Date of publication of application: 19.10.2022
(73) Proprietor: Gambro Lundia AB, 226 43 Lund (SE)
(72) Inventor: VERNIN, Guillaume, 69330 Meyzieu (FR)
(74) Representative: PGA S.p.A., Milano, Succursale di Lugano
(86) International application number: PCT/EP2020/076073
(87) International publication number: WO 2021/115651

(56) References cited:
- WO-A2-03/006944
- WO-A2-2007/008448
- FR-A1- 2 889 451
- US-A1- 2011 230 772
- US-A1- 2013 110 028

## Description

### TECHNICAL FIELD

The invention relates to a fluid diverting device for an apparatus for extracorporeal treatment of blood and to a blood set for an apparatus for extracorporeal treatment of blood provided with this fluid diverting device.

Extracorporeal blood treatment involves removing blood from a patient, treating the blood externally to the patient, and returning the treated blood to the patient. Extracorporeal blood treatment is typically used to extract undesirable matter or molecules from the patient's blood and add desirable matter or molecules to the blood. Extracorporeal blood treatment is used with patients unable to effectively remove matter from their blood, such as when a patient has suffered temporary or permanent kidney failure. These patients and other patients may undergo extracorporeal blood treatment to add or remove matter to their blood, to maintain an acid/base balance or to remove excess body fluids, or to perform extracorporeal gas exchange processes, for example.

For instance, in a haemodialysis treatment a patient's blood and a treatment liquid approximately isotonic with blood flow are circulated in a respective compartment of haemodialyser, so that, impurities and undesired substances present in the blood (urea, creatinine, etc.) may migrate by diffusive transfer from the blood into the treatment liquid. The ion concentration of the treatment liquid is chosen so as to correct the ion concentration of the patient's blood. In a treatment by haemodiafiltration, a convective transfer by ultrafiltration, resulting from a positive pressure difference created between the blood side and the treatment-liquid side of the membrane of a haemodiafilter, is added to the diffusive transfer obtained by dialysis. Hemoperfusion, oxygenation or decarboxylation treatments are also known.

A vascular access is used to remove the patient's blood so that it is filtered through the blood treatment unit (dialyzer, filter) and then returned to the patient. In case of chronic treatments, the vascular access may be arterio-venous. Two needles may be inserted into the vein, one to draw blood and one to return it. The orientation of the needles takes the normal flow of the blood into account. The "arterial" needle draws blood from the "upstream" location while the "venous" needle returns blood "downstream". In case of acute treatments, the vascular access may be veno-venous, i.e. using a double-lumen catheter inserted in a central vein. The blood treatment unit comprises pressure monitoring devices, as to check, for example, for circuit obstructions.

### BACKGROUND OF THE INVENTION

In the field of extracorporeal blood treatments and therapies, some problems reported by users are issues at the vascular access. Access Extremely Negative (AEN) is one of the most encountered alarm in the field, mainly linked to catheter tip stuck against blood vessel's wall or, more rarely, to clots obstructing the access way of the catheter. When this alarm relates to catheter positioning inside the vein, the nurse has to get the access pressure back closer to an atmospheric level, e.g. through the use of a Y-connector connected to the access line, unstick the catheter tip from the vein wall and then try to move the catheter inside the vein and resume the treatment.

Another drawback is that at the end of the treatment, when blood is to be returned to the patient, one operator has to disconnect the patient access and connect a saline bag to the set's access line, in aseptic conditions, and another operator has to operate on the monitor of the machine in non-aseptic conditions. Therefore, two operators are needed for one patient.

Another drawback is that, as per the blood return described above, both saline and blood recirculation procedures currently require sterile and non-sterile interventions and the manual connection of supplementary devices (Y-connector, saline bag).

In all the cases detailed above, the operator has to handle the vascular access, to disconnect and eventually reconnect the same to the patient. All this implies operator workload and increased probability of occurrence for non-aseptic handling.

Furthermore, the use of a Y-connector connected to the access line involves further risks. On the access side, the user may forget to clamp the Y saline line prior to resuming the treatment. In case of blood pump stop and slightly negative patient access pressure, the saline bag content would be sucked into the catheter, causing air ingress leading to set clotting (or potentially air embolism). Additional modules, configured to be placed in-line between the blood treatment apparatus and the patient vascular access and configured to divert the flow of blood without necessarily disconnecting the patient, are also known.

For instance, document US 3, 626, 938 discloses a shunt valve device which is designed to be mounted permanently on a body member of a person and permanently connected to the artery and vein of the person. The device is provided with valve means for selectively connecting the artery and vein to an artificial kidney or the like. The device further has rotary shunt means for directing the flow of blood back to the body member when the device is not operatively connected to the artificial kidney.

Document US 5,894,011 discloses a device for selectively controlling the direction of blood flow to and from the patient during hemodialysis. The device comprises two interlocking disks that rotate in relation to each other without separating. The two disks have fluid fittings that allow the blood lines attached to the patient to connect to one of the disks and the blood inlet and outlet for the hemodialysis machine to connect to the other. The center of each fluid fitting is a channel that aligns to a corresponding channel in the other disk. The disks rotate between two fixed relative positions, referred to herein as preferred alignments. The preferred alignments are such that the line drawing blood from the patient in the first preferred alignment becomes the line returning blood to the patient in the second preferred alignment, and the line returning blood to the patient in the first preferred alignment becomes the line drawing blood from the patient in the second preferred alignment. A bypass channel allows blood to flow from the outlet to the inlet of the hemodialysis machine when the device is in neither of its two preferred alignments.
DocumentUS20110230772A1 discloses a device and method for invasive blood pressure measurement in a vascular access under continuous blood flows in a treatment device in extracorporeal detoxification methods. Systemic arterial pressure is determined directly or indirectly. A valve-controlled bypass system which goes around a blood pumping unit is provided so that the blood flow in the treatment device is not interrupted and alarms are suppressed. DocumentUS20130110028A1 discloses a valve arrangement for use in an extracorporeal blood circuit. The valve arrangement has a first valve arranged in the arterial blood line, a second valve arranged in the venous blood line, a fourth valve arranged in a first arteriovenous connection line between the arterial blood line and the venous blood line and a fifth valve arranged in a second arteriovenous connection line between the arterial blood line and the venous blood line and/or a third valve arranged for establishing a fluid connection in a blood line between the arterial and the venous blood lines and/or a sixth valve arranged between a blood treatment device and an air venting device. Document FR 2889451A1 discloses an extracorporeal fluid circulating device for realizing hemodialysis operation, comprising two tubes each having ends connected to one of two channels of a catheter and ends connected to one of two channels of a dialysis machine. Junctions are placed between the ends of the tubes, where two of the junctions are connected to the other two junctions by additional tubes. A closing unit closes simultaneously the tubes or two portions of the tubes each situated between the junctions. Document WO 2007/008448 discloses a method for monitoring for leaks or disconnections, comprising the steps of operating a blood pump to circulate blood through an extracorporeal blood circuit, opening a shunt connection between the arterial and venous blood flow portions, sensing the presence of air from any leaks or disconnections within the venous blood flow portion and taking corrective action if the presence of air is noted.

These devices allow a limited number of flow path configurations and, due to their structure and geometry of the ducts, may cause pressure drops and/or clotting.

It is therefore an object of the present invention to provide a fluid diverting device for an apparatus for extracorporeal treatment of blood, wherein the fluid diverting device is configured to divert the flow of blood or other fluid/s in order to perform a plurality of procedures (e.g. all the steps of the treatment, pre and post treatment and possible troubleshooting procedures) without necessarily disconnecting the patient.

It is an object of the present invention to provide a fluid diverting device which may be easily interposed between the apparatus for extracorporeal treatment of blood and the patient, featuring various functional components and able to be set in a plurality of configurations to perform the plurality of procedures.

In particular, it is an object providing a fluid diverting device able to provide easy and safe AEN troubleshooting, blood return at the treatment end, blood recirculation, saline recirculation.

It is a further object providing a fluid diverting device allowing to control its configurations in automated manner.

It is also an object of the present invention to provide a fluid diverting device enabling offering the device features as a supplemental option with respect to the apparatus.

It is a further object providing a fluid diverting device allowing a single person to handle the patient's vascular access in safe manner and in aseptic conditions.

It is a further object providing a fluid diverting device offering the possibility to have some commands located conveniently nearby the patient access.

It is a further object providing a fluid diverting device offering the possibility to operate disinfection procedures in aseptic conditions.

It is a further object providing a fluid diverting device which is structurally simple, cost effective and reliable.

It is a further object providing a fluid diverting device able to prevent clotting and/or to avoid high pressure losses and/or requiring minimal amounts of fluid needed for flushing said fluid diverting device.

### SUMMARY

At least one of the above objects is substantially reached by a fluid diverting device for an apparatus for extracorporeal treatment of blood and by an apparatus for extracorporeal treatment of blood according to one or more of the appended claims.

A fluid diverting device for an apparatus for extracorporeal treatment of blood, an apparatus for extracorporeal treatment of blood and a method for diverting a flow of liquid and/or blood in an apparatus for extracorporeal treatment of blood capable of achieving one or more of the above objects are here below described.

A 1^{st} aspect concerns a fluid diverting device for an apparatus for extracorporeal treatment of blood according to claim 1.

A 2^{nd} aspect concerns a blood set for an apparatus for extracorporeal treatment of blood according to claim 15.

An apparatus for extracorporeal treatment of blood is described comprising the blood set of the previous aspect.

A method for diverting a flow of liquid and/or blood in an apparatus for extracorporeal treatment of blood without disconnecting the patient is also described.

### DESCRIPTION OF THE DRAWINGS

The attached drawings, which are provided by way of non-limiting example, wherein:
Figure 1 shows a schematic diagram of an apparatus for extracorporeal treatment of blood comprising a fluid diverting device;
Figure 2 shows an enlarged view of the fluid diverting device of Figure 1;
Figure 3 shows another embodiment of the fluid diverting device
Figure 4 shows a variant of the fluid diverting device of Figure 2;
Figures 5 to 11 show respective working configurations of the fluid diverting device of Figure 1;
Figures 12 to 15 show flowcharts of respective operating modes of the of the fluid diverting device;
Figure 16 shows an apparatus for extracorporeal treatment of blood comprising a fluid diverting device according to the invention;
Figures 17 to 19 show respective working configurations of the fluid diverting device of Figure 16;
Figures 20A and 20B show enlarged views of an element of the fluid diverting device of Figure 16 in respective working positions;
Figure 21 shows a variant of the apparatus for extracorporeal treatment of Figure 16;
Figures 22 to 25 show respective working configurations of the fluid diverting device of Figure 21;
Figure 26 is a flowchart of a respective operating mode of the of the apparatus of Figures 21 to 25;
Figure 27 shows an apparatus for extracorporeal treatment of blood comprising a different embodiment of a fluid diverting device;
Figures 28A and 28B show enlarged views of an element of the fluid diverting device of Figure 27 in respective working positions;
Figures 29 and 30 show respective working configurations of the fluid diverting device of Figure 27;
Figure 30 is a flowchart of a respective operating mode of the apparatus of Figures 27 to 30;
Figure 31 is a flowchart of another operating mode of the apparatus of Figures 27 to 30.

### DETAILED DESCRIPTION

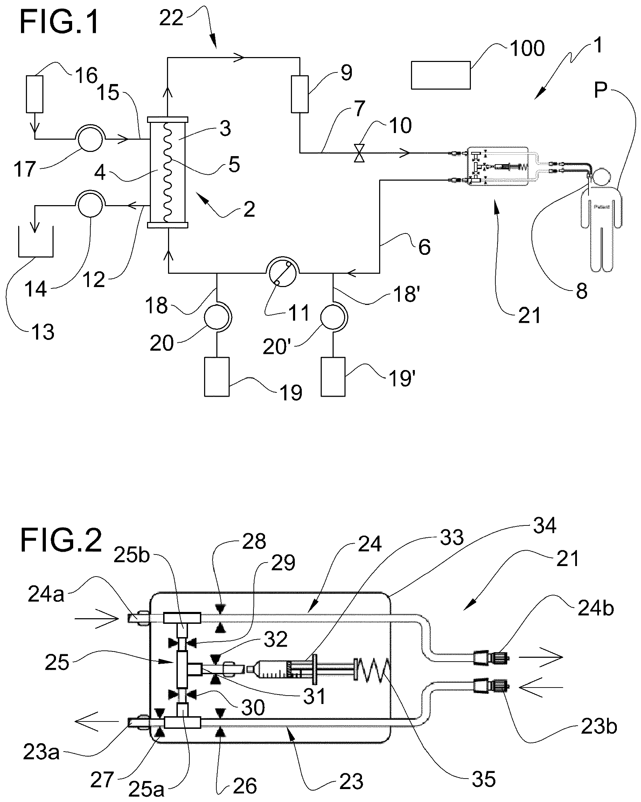

Non-limiting embodiments of a fluid diverting device for an apparatus 1 for extracorporeal treatment of blood are shown in Figures 2, 3, 4, 16, 21 and 26. In below description and in Figures 2, 3, 4, 16, 21 and 26 same components are identified by same reference numerals.

An apparatus for the extracorporeal treatment of blood 1 is represented in Figure 1. The apparatus 1 of Figure 1 comprises a blood treatment unit 2 (such as a hemofilter, an ultrafilter, an hemodiafilter, a dialyzer, a plasmafilter and the like) having a primary chamber 3 and a secondary chamber 4 separated by a semipermeable membrane 5. Depending upon the treatment, the semipermeable membrane 5 of the blood treatment unit 2 may be selected to have different properties and performances.

A blood withdrawal line 6 is connected to an inlet of the primary chamber 3 and a blood return line 7 is connected to an outlet of the primary chamber 3. In use, the blood withdrawal line 6 and the blood return line 7 are connected to a needle or to a catheter or other access device 8 which is then placed in fluid communication with the patient "P" vascular system, such that blood may be withdrawn through the blood withdrawal line 6, flown through the primary chamber 3 and then returned to the patient's vascular system through the blood return line 7. An air separator, such as a deaeration chamber 9, may be present on the blood return line 7. Moreover, a monitor valve 10 may be present on the blood return line 7, downstream the deaeration chamber 9.

The blood flow through the blood lines is controlled by a blood pump 11, for instance a peristaltic blood pump, acting either on the blood withdrawal line 6 or on the blood return line 7. The embodiment of Figure 1 shows the blood pump 11 coupled to a pump section of the withdrawal line 6. A control unit 100 is connected and controls the blood pump 11 to regulate a blood flow rate.

An effluent fluid line 12 or spent dialysate line may be connected, at one end, to a fluid outlet of the secondary chamber 4 and, at its other end, to a waste which may be a discharge conduit or an effluent fluid container 13 collecting the effluent fluid extracted from the secondary chamber 4. An effluent pump 14 that operates on the effluent fluid line 13 under the control of the control unit 100 to regulate a flow rate of the effluent fluid through the effluent fluid line.

The apparatus of Figure 1 includes a dialysis fluid line 15 connected at one end with a fresh dialysis liquid source 16 and at its other end with a fluid inlet of the secondary chamber 4 of the treatment unit 2 for supplying fresh dialysis liquid to the secondary chamber 4. A dialysis fluid pump 17 is operative on the dialysis fluid line 15 under the control of the control unit 100 to supply fluid from the dialysis liquid source 16 to the secondary chamber 4 and to regulate the flow rate of the dialysis liquid.

The embodiment of Figure 1 further presents an infusion line 18 connected to the blood withdrawal line 6 between the blood pump 11 and the treatment unit 2. This infusion line 18 supplies replacement fluid from an infusion fluid container 19 connected at one end of the infusion line 18. Note that, alternatively or in addition to the infusion line 18, the apparatus of Figure 1 may include a post-dilution fluid line (not shown) connecting an infusion fluid container to the blood return line 7 and/or a pre-blood pump infusion line 18' with its own pre-blood pump infusion fluid container 19'. Furthermore, an infusion pump 20 operates on the infusion line 18 and an infusion pump 20' operates on the pre-blood pump infusion line 18' to regulate respective flow rates through the infusion lines 18, 18'. Also the infusion pumps 20, 20' are operative under the control of the control unit 100.

The effluent fluid line 12, the dialysis fluid line 15 and the secondary chamber 4 of the blood treatment unit 2 are part of a fluid circuit of the apparatus 1. The blood withdrawal line 6, the blood return line 7, the primary chamber 3 of the treatment unit 2 form part of an extracorporeal blood circuit of the apparatus 1. The infusion lines 18, 18' form part of an infusion circuit of the apparatus 1.

The control unit 100 may comprise a digital processor (CPU) with memory (or memories), an analogical type circuit, or a combination of one or more digital processing units with one or more analogical processing circuits. In the present description and in the claims it is indicated that the control unit 100 is "configured" or "programmed" to execute steps: this may be achieved in practice by any means which allow configuring or programming the control unit 100. For instance, in case of a control unit 100 comprising one or more CPUs, one or more programs are stored in an appropriate memory: the program or programs containing instructions which, when executed by the control unit 100, cause the control unit 100 to execute the steps described and/or claimed in connection with the control unit 100. Alternatively, if the control unit 100 is of an analogical type, then the circuitry of the control unit 100 is designed to include circuitry configured, in use, to process electric signals such as to execute the control unit 100 steps herein disclosed.

The control unit 100 may also be operatively connected to sensors (like flow sensors and/or pressure sensors) on the blood circuit and/or fluid circuit and/or infusion circuit. The control unit 100 is also operatively connected to clamps and valves, like the monitor valve 10. The control unit 100 may also be connected to a user interface, not shown, for instance a graphic user interface, which receives operator's inputs and displays the apparatus outputs. For instance, the graphic user interface may include a touch screen, a display screen and hard keys for entering user's inputs or a combination thereof. During extracorporeal blood treatment, the control unit 100 is configured to control at least the pumps 11, 14, 17, 20, 20' to make sure that a prefixed patient net fluid removal is achieved in the course of a treatment time, as required by a prescription provided to the control unit 100, e.g. via the user interface.

The apparatus 1 comprises a main portion 22 comprising at least a casing for the control unit 100, the control unit 100, the user interface, the blood pump 11, the effluent pump 14, the dialysis fluid pump 17, the infusion pumps 20, 20' and devices to mount a disposable set (comprising the blood treatment unit 2, the extracorporeal blood circuit, the infusion circuit and the fluid circuit) to the main portion 22.

The apparatus for the extracorporeal treatment of blood 1 shown in Figure 1 further comprises a fluid diverting device 21 which is configured to be placed in-line between the main portion 22 of the apparatus 1 and the vascular access of a patient "P", in particular the access device 8 (e.g. a needle or a catheter) placed in fluid communication with the patient "P" vascular system. The fluid diverting device 21 may be placed in-line on the withdrawal line 6 and on the return line 7 and closer to the access device 8 than to the main portion 22 of the apparatus 1 for extracorporeal treatment of blood. The fluid diverting device 21 may be disposable, at least in part. The fluid diverting device 21 of Figure 1 is an add-on module which may be supplied to the apparatus and connected to the withdrawal line 6 and return line 7 at a later stage with respect to the manufacturing of the apparatus 1.

The fluid diverting device 21 comprises a substantially H-shaped conduits assembly comprising a withdrawal conduit 23, a return conduit 24 and a bridging conduit 25 connecting the withdrawal conduit 23 to the return conduit 24. Each of the withdrawal conduit 23, the return conduit 24 and the bridging conduit 25 of the embodiments shown in the annexed Figures is a straight plastic tube.The withdrawal conduit 23 is parallel to the return conduit 24 while the bridging conduit 25 is perpendicular to the withdrawal conduit 23 and to the return conduit 24.

The withdrawal conduit 23 has a first extremity 23a and an opposite second extremity 23b each provided with a quick connector, such as a Luer connector, to allow easy and quick connection/disconnection to/from the remaining portions of the withdrawal line 6. The first extremity 23a is connected or configured to be connected to a portion of the withdrawal line 6 comprising the pump section coupled to the blood pump 11. The second extremity 23b is connected or configured to be connected to a portion of the withdrawal line connected to the access device 8. The return conduit 24 has a first extremity 24a and an opposite second extremity 24b each provided with a quick connector, such as a Luer connector, to allow easy and quick connection/disconnection to/from the remaining portions of the return line 7. The first extremity 24a is connected or configured to be connected to a portion of the return line 7 comprising the deaeration chamber 9 and the monitor valve 10. The second extremity 24b is connected or configured to be connected to a portion of the return line 7 connected to the access device 8.

The bridging conduit 25 has a first extremity 25a forming a junction to the withdrawal conduit 23 and an opposite second extremity 25b forming a junction to the return conduit 24. For instance, the bridging conduit 25, the withdrawal conduit 23 and the return conduit 24 are connected through T-connectors. The withdrawal conduit 23 comprises two branches connected to the T-connector. A branch comprising the first extremity 23a and a branch comprising the second extremity 23b. The return conduit 24 comprises two branches connected to the T-connector. A branch comprising the first extremity 24a and a branch comprising the second extremity 24b.

A plurality of valves (e.g. clamps) operate on the withdrawal conduit 23, on the return conduit 24 and on the bridging conduit 25 and are configured to divert a flow of liquid and/or blood without disconnecting the patient "P".

The fluid diverting device 21 of the embodiments of Figures 1, 2 and 4 to 11 comprises a first withdrawal valve 26 and a second withdrawal valve 27 operating on the withdrawal conduit 23. The first withdrawal valve 26 is placed upstream, with respect to a flow of blood during treatment (flowing from the second extremity 23b to the first extremity 23a of the withdrawal conduit 23), the junction of the bridging conduit 25 to said withdrawal conduit 23 and the second withdrawal valve 27 is placed downstream, with respect to said flow of blood during treatment, the junction of the bridging conduit 25 to said withdrawal conduit 23. As disclosed in Figure 2, the first withdrawal valve 26 and the second withdrawal valve 27 are placed on opposite sides with respect to the T-connector joining the bridging conduit 25 to the withdrawal conduit 23.

The fluid diverting device 21 of the embodiments of Figures 1, 2 and 4 to 11 comprises a single return valve 28 operating on the return conduit 24. The return valve 28 is placed downstream the junction of the bridging conduit 25 to the return conduit 24 with respect to a flow of blood during treatment (flowing from the first extremity 24a to the second extremity 24b of the return conduit 24). As disclosed in Figure 2, the return valve 28 is placed at a side of the T-connector joining the bridging conduit 25 to the return conduit 24 and on the branch comprising the second extremity 24b of said return conduit 24.

In a variant embodiment, not shown, a first return valve and a second return valve operate on the return conduit 24 and are placed on opposite sides with respect to the T-connector joining the bridging conduit 25 to the return conduit 24, similar to the first withdrawal valve 26 and the second withdrawal valve 27. The return valve placed upstream the junction of the bridging conduit 25 to the return conduit 24 with respect to the flow of blood during treatment may perform the function of the monitor valve 10. The fluid diverting device 21 of the embodiments of Figures 1, 2 and 4 to 11 comprises a first bridging valve 29 and a second bridging valve 30 operating on the bridging conduit 25. The first bridging valve 29 is placed closer to the return conduit 24 (closer than the second bridging valve 30) and the second bridging valve 30 is placed closer to the withdrawal conduit 23 (closer than the first bridging valve 29).

The bridging conduit 25 comprises a first branch having the first extremity 25a and a second branch having the second extremity 25b. The first branch and the second branch are connected to each other through a T-connector delimiting also an access point 31 suitable to connect a liquid source. The first bridging valve 29 is placed on the second branch bridging conduit 25 and the second bridging valve 30 is placed on the first branch of the bridging conduit 25. The access point 31 is located between the first bridging valve 29 and the second bridging valve 30.

A stretch of tubing departs form the access point 31 and is provided with an access valve 32. The stretch of tubing may be connected to a liquid source 33, like a syringe, e.g. of 50 ml, or a bag, e.g. of 500 ml. The liquid of said liquid source may be saline.

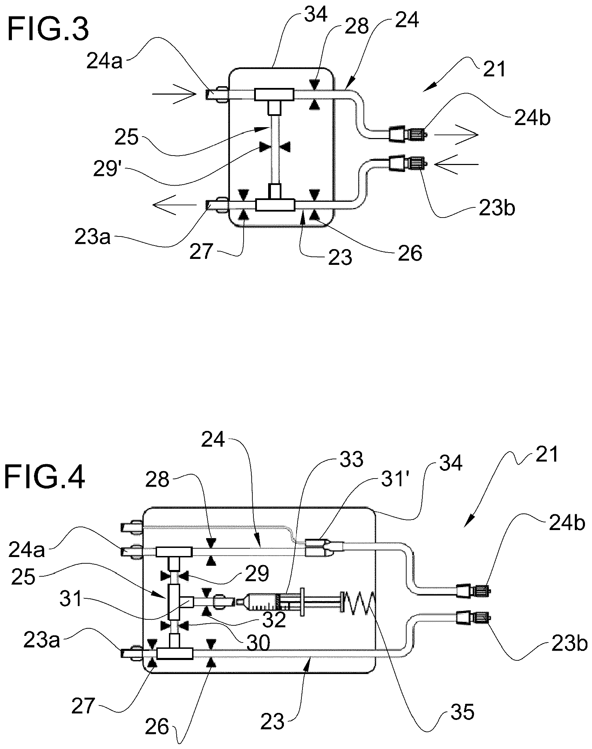

In the variant embodiment of Figure 3, the fluid diverting device 21 comprises a single bridging valve 29' operating on the bridging conduit 25 and does not comprise any access point 31. In the variant embodiment of Figure 4, the fluid diverting device 21 further comprises (in addition with respect to the embodiment of Figure 3) a further access point 31' on the return conduit 24, e.g. for calcium infusion. The further access point 31' is placed on the branch comprising the second extremity 24b and may be connected to a bag of the apparatus.

The substantially H-shaped conduits assembly and the plurality of valves 26, 27, 28, 29, 30, 32 are mounted on a holder 34 which may be shaped like a plate provided with seats, or other holding devices, configured to accommodate the substantially H-shaped conduits assembly and the plurality of valves 26, 27, 28, 29, 30, 32. In the embodiments of Figures 1, 2 and 4 to 11, the holder 34 comprises a seat, or other holding device to hold the syringe 33, and a spring 35 for pressurizing the syringe 33 and ejecting the fluid contained therein through the access point 31.

The fluid diverting device 21 may also comprise one or more sensors (e.g. pressure sensor, flowmeter, air detector, etc.), not shown, active on the H-shaped conduits assembly, i.e. on the return conduit 24 and/or on the withdrawal conduit 23, on the bridging conduit 25.

The plurality of valves 26, 27, 28, 29, 30, 32 and the sensor/s are operatively connected or connectable to the control unit 100 of the apparatus 1 and/or to a control unit of the fluid diverting device 21 which may be slaved to the control unit 100 of the apparatus 1. Said control unit/s is/are configured for commanding, optionally automatically, the configurations of the plurality of valves 26, 27, 28, 29, 30, 32. The valves 26, 27, 28, 29, 30, 32 and/or the control unit of the fluid diverting device 21 may be connected to the control unit 100 of the apparatus 1 through a wired or wireless communication device.

The fluid diverting device 21 comprises a power supply to power the valves 26, 27, 28, 29, 30, 32 and/or the sensor/s and/or control unit or said valves 26, 27, 28, 29, 30, 32 and/or the control unit of the fluid diverting device 21 may be connected to a power supply of the apparatus 1 to be powered by said apparatus 1.

The fluid diverting device 21 may be a portable module and/or comprises an attachment device configured to install said fluid diverting device on another element, optionally in a removable manner, e.g. to clothing of a patient "P" or to a bed for the patient "P" or to a main portion of the apparatus for extracorporeal treatment of blood. The lengths of the portion of the return line 7 comprising the deaeration chamber 9 and the monitor valve 10 and of the portion of the withdrawal line 6 comprising the pump section coupled to the blood pump 11 are such to place the fluid diverting device close to the patient "P".

In use and according to a method for diverting a flow of liquid and/or blood in the apparatus 1, before treatment of the patient "P", the blood circuit is primed and the control unit 100 controls the valves 26, 27, 28, 29, 30, 32 to allow priming the withdrawal conduit 23, the return conduit 24 and the bridging conduit 25, using either solution bags of the apparatus 1 or saline from the liquid source 33 connected to the access point 31.

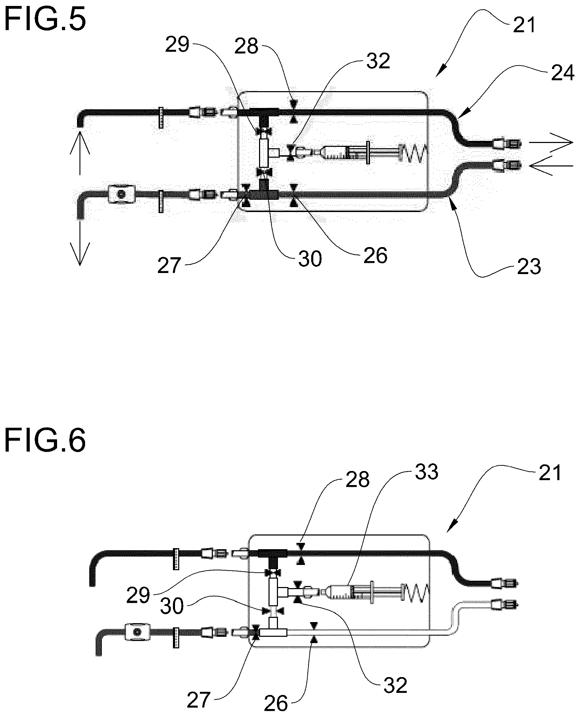

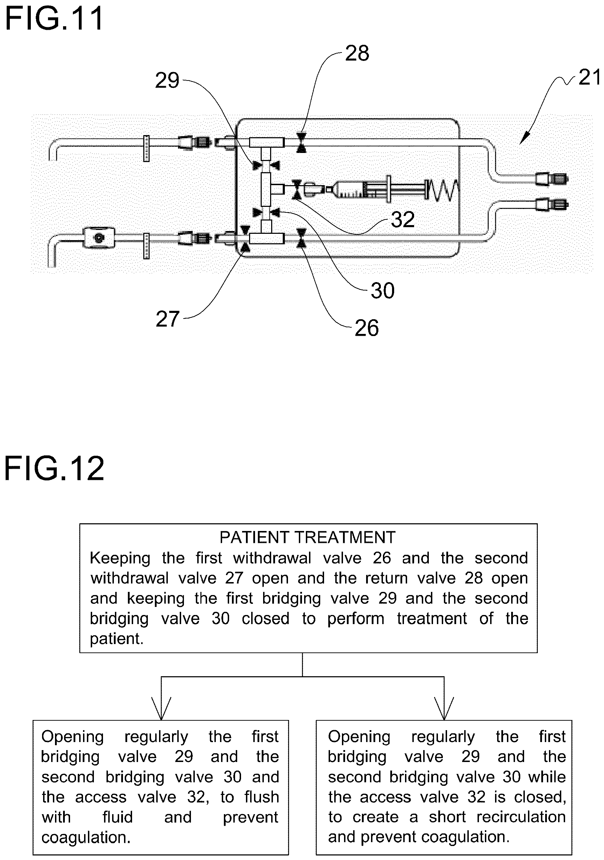

During patient treatment (Figures 5 and 12), when blood flows from patient "P" through the withdrawal line 6, into the primary chamber 3 of the blood treatment unit 2 and then back into the patient "P" through the return line 7, the control unit 100 controls the valves 26, 27, 28, 29, 30, 32 to configure said valves 26, 27, 28, 29, 30, 32 in a treatment configuration. In the treatment configuration, the first bridging valve 29, the second bridging valve 30 and the access valve 32 are closed, the first withdrawal valve 26, the second withdrawal valve 27 and the return valve 28 are open.

During patient treatment, since blood may stagnate at the closed first bridging valve 29 and the second bridging valve 30, in order to prevent coagulation, the first bridging valve 29 and the second bridging valve 30 and the access valve 32 are opened regularly (intermittently), to flush the bridging conduit 25 with fluid and prevent coagulation. According to a variant of the method, the first bridging valve 29 and the second bridging valve 30 are opened regularly (intermittently) while the access valve 32 is closed, to flush the bridging conduit 25 with blood, creating a short recirculation and preventing coagulation (Figure 12).

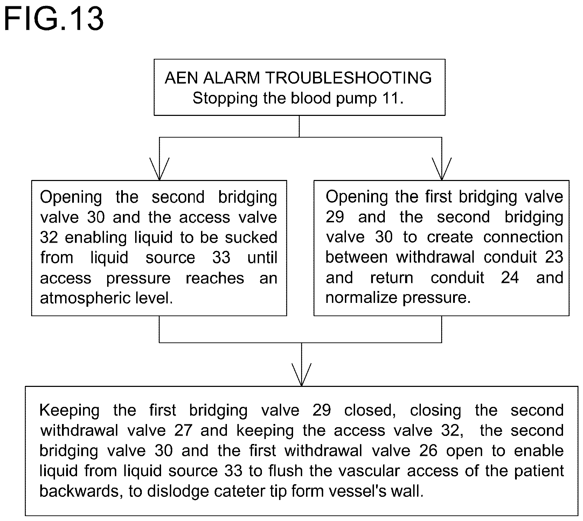

In case of Access Extremely Negative (AEN) alarm, the blood pump 11 is stopped, the second bridging valve 30 and the access valve 32 are opened to enable saline to be sucked from the syringe 33 until access pressure reaches an approximately atmospheric level or the first bridging valve 29 and the second bridging valve 30 are both opened to create connection between the withdrawal conduit 23 and the return conduit 24 and to normalize pressure. After the access pressure got closer to the atmospheric level, the first bridging valve 29 is kept closed, the second withdrawal valve 27 is closed while the access valve 32, the second bridging valve 30 and the first withdrawal valve 26 are kept open. This way, the fluid from syringe 33 flushes the vascular access of the patient "P" backwards to help dislodge the catheter tip from vessel's wall (Figures 6 and 13).

According to another possible action to solve an AEN alarm, the first withdrawal valve 26 and the second withdrawal valve 27 are closed while the other valves are open. The blood pump 11 is controlled to turn in order to increase the access negative pressure till a defined value and then stopped. Then the first withdrawal valve 26 and the second withdrawal valve 27 are opened to create a sudden negative pressure peak in the catheter's access way to move or break a clot.

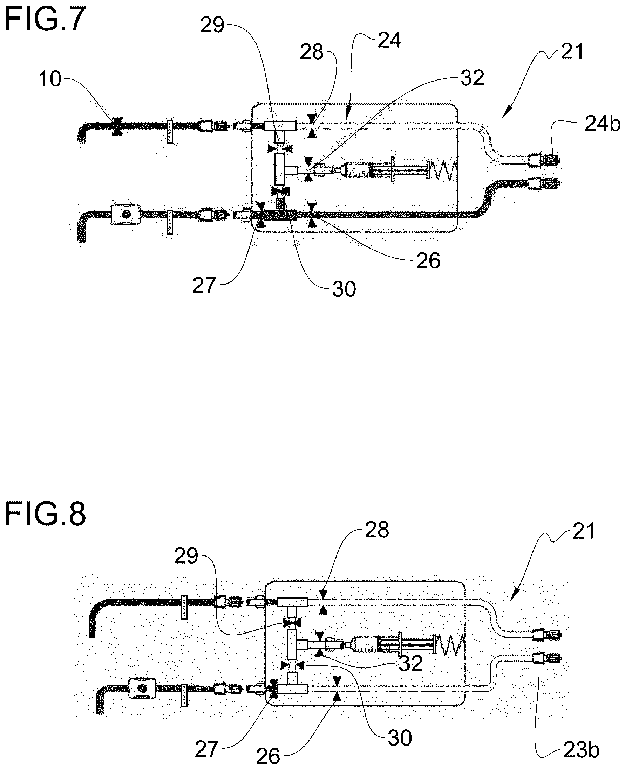

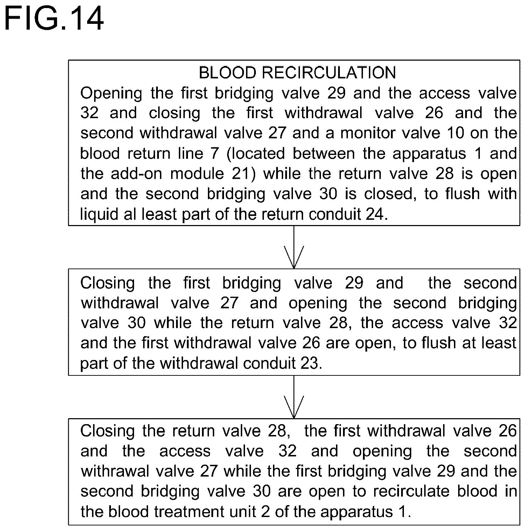

If these attempts fail, the control unit 100 controls the plurality of valves to 26, 27, 28, 29, 30, 32 to engage blood recirculation, while notifying the staff that action is required (moving the patient "P", caring for the vascular access, etc.). Prior to enabling blood recirculation, flushing is needed in the return conduit 24 and withdrawal conduit 23 to avoid clotting and to enable patient reconnection after staff intervention. To this aim, the branch of the return conduit 24 comprising the second extremity 24b is flushed with fluid by closing the second bridging valve 30 and the monitor valve 10 while the first bridging valve 29, the first withdrawal valve 26, the second withdrawal valve 27, the return valve 28 and the access valve 32 are open and fluid from the syringe 33 is injected (Figures 7 and 14).

The branch of the withdrawal conduit 6 having the second extremity 23b is then flushed. The first bridging valve 29 and the second withdrawal valve 27 are closed, the second bridging valve 30, the first withdrawal valve 26 and the access valve 32 are open and saline from the syringe 33 is injected (Figures 8 and 14).

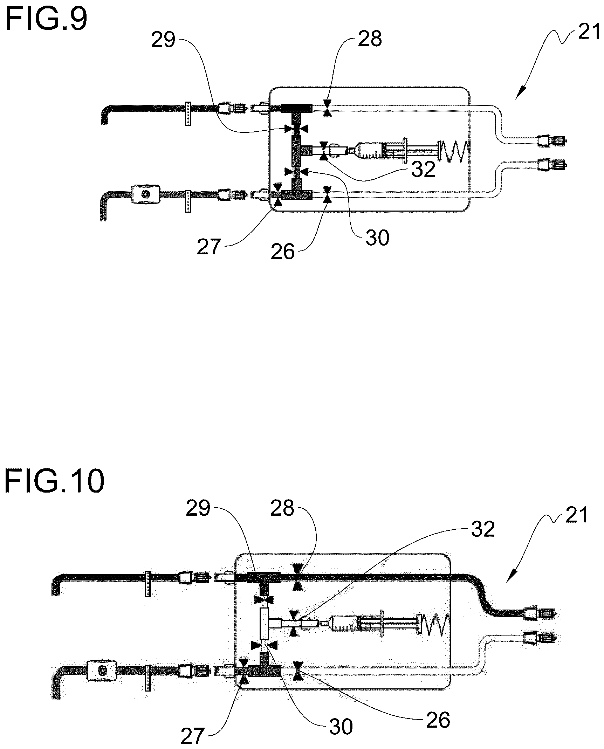

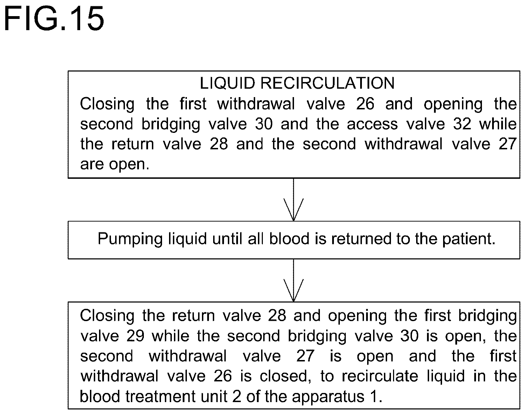

The blood recirculation starts with the return valve 28 closed, the first withdrawal valve 26 closed, the second withdrawal valve 27 open and the first and second bridging valves 29, 30 open, so that blood recirculates in the blood treatment unit 2 of the apparatus 1 and staff intervention is executed (Figures 9 and 14). At the end of the treatment, with blood return to the patient "P", saline recirculation starts. With the first withdrawal valve 26 and the first bridging valve 29 closed and the second bridging valve 30, the return valve 28, the second withdrawal valve 27 and the access valve 32 open, saline is pumped by the blood pump 11 until all blood is returned to the patient "P". Then, the return valve 28 is closed and the first bridging valve 29 is opened while the second bridging valve 30 is still open, the second withdrawal valve 27 is open and the first withdrawal valve 26 is closed, to recirculate liquid in the blood treatment unit 2 of the apparatus 1 (Figures 11 and 15).

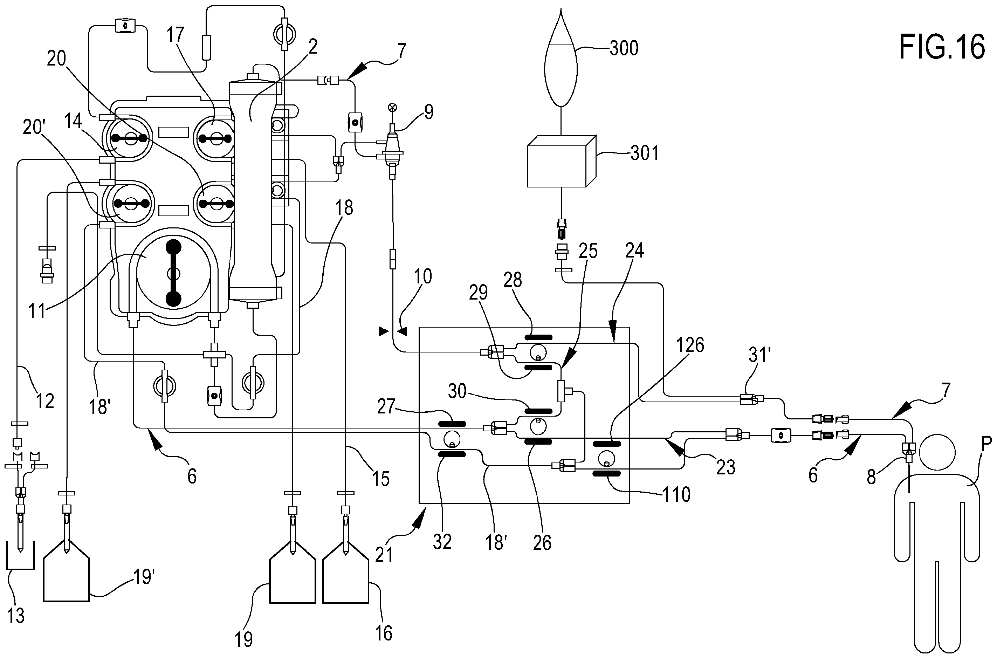

Figures 16 to 20B show an embodiment of the fluid diverting device 21 according to the invention. The fluid diverting device 21 of this embodiment may be mounted on a side of the main portion 22 of the apparatus 1.

The fluid diverting device 21 comprises the first withdrawal valve 26, the second withdrawal valve 27, the return valve 28, the first bridging valve 29, the second bridging valve 30 and the access point 31 as the embodiment of Figures 1, 2 and 4 -11.

The type of valves is different from the embodiment of Figures 1, 2 and 4 -11. Each of the plurality of valves is a rotating clamp or pinch.

The first bridging valve 29 and the return valve 28 share a common rotating body 140 and comprise two respective abutment elements 145, one for the first bridging valve 29 and one for the return valve 28. The second bridging valve 30 and the first withdrawal valve 26 share a common rotating body 140 and two respective abutment elements 145, one for the second bridging valve 30 and one for first withdrawal valve 26.

Figure 16 also shows a calcium bag 300 connected to the further access point 31' on the return conduit 24 for calcium infusion operated through a calcium pump 301.

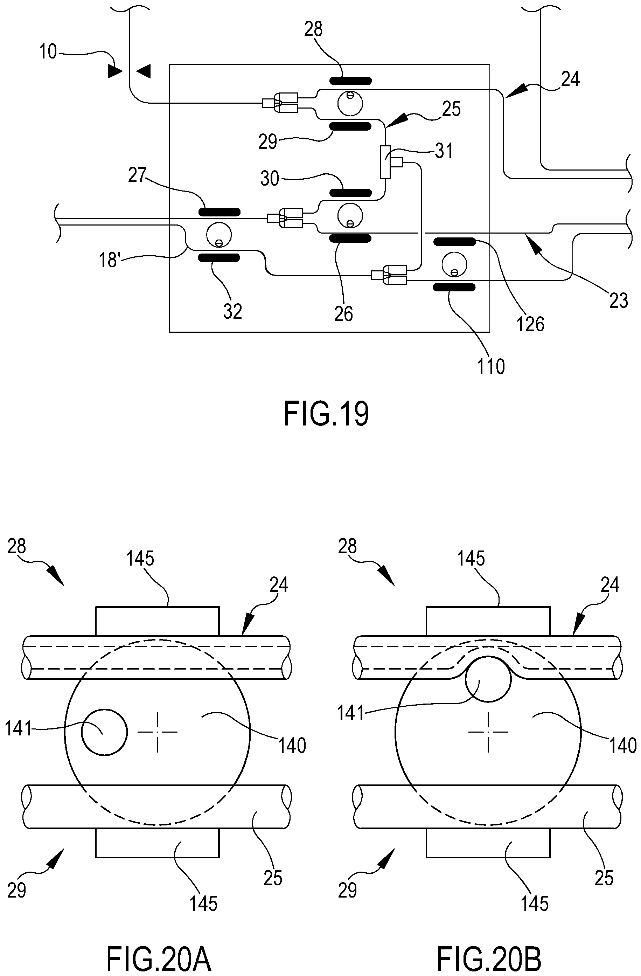

Figures 20A and 20B show an enlarged view of the first bridging valve 29 and the return valve 28. Each valve comprises the rotating body 140 and one abutment element 145. The rotating body 140 comprises a presser 141 spaced form a rotation axis of the rotating body 140. The presser 141 is placed close to the rotating body 140 such that a tube section may be positioned between the abutment element 145 and the presser 141. The assembly comprising the first bridging valve 29 and the return valve 28 shown in

Figures 20A and 20B comprises one rotating body 140 and two abutment elements 145. Said two abutment elements 145 are stationary with respect to the rotation axis of the rotating body 140 and are positioned on opposite sides with respect to the rotating body 140. A tube section of the return conduit 24 is placed against one of the abutment elements 145 and a tube section of the bridging conduit 25 is placed against the other of the abutment elements 145.

The rotating body 140 is rotatable between a first clamping position (one of the two valves is closed and the other is open), a second clamping position (one of the two valves is closed and the other open) and a release position (both valves are open).

In the first clamping position (Figure 20B), the presser 141 of the rotating element 140 is close to one of the two abutment elements 145 to press and close a portion of a tube (the return conduit 24 in Figure 20B) between said rotating element 140 and said abutment element 145.

In the second clamping position (not show), the presser 141 of the rotating element 140 is close to the other of the two abutment elements 145 to press and close a portion of another tube (it should be the bridging conduit 25 in Figures 20A and 20B) between said rotating element 140 and said abutment element 145.

In the release position, the presser 141 of the rotating element is spaced from both the abutment elements 145, to open both portions of tubes (Figure 20A).

The assembly comprising the first withdrawal valve 26 and the second bridging valve 30 has the same structure and functionality shown in Figures 20A and 20B and disclosed above.

The access point 31 of this embodiment is connected to the pre-blood pump infusion line 18' with its own pre-blood pump infusion fluid container 19' and infusion pump 20'. The pre-blood pump infusion line 18' has a branch connected to the access point 31 and a branch connected to the blood withdrawal line 6 at a site between the vascular access 8 and the fluid diverting device 21. The infusion pump 20' operates on the pre-blood pump infusion line 18' to regulate respective flow rates through the cited branches. A first infusion valve or access valve 32 is placed upstream the branch of the pre-blood pump infusion line 18' connected to the access point 31. A second infusion valve 110 is placed downstream the branch connected to the access point 31, i.e. on the branch connected to the blood withdrawal line 6.

Also the access valve 32 is a rotating clamp or pinch. Like the assembly comprising the first withdrawal valve 26 and the second bridging valve 30 and the assembly comprising the first bridging valve 29 and the return valve 28, also the access valve 32 is coupled to the second withdrawal valve 27. The second withdrawal valve 27 and the access valve 32 comprise one common rotating body 140 and two respective abutment elements 145.

An auxiliary withdrawal valve 126 operates on the withdrawal conduit 6 and is placed downstream, with respect to a flow of blood during treatment, the first withdrawal valve 26. The second infusion valve 110 and the auxiliary withdrawal valve 126 comprise one common rotating body 140 and two respective abutment elements 145.

As shown in Figures 16 to 20B, the common rotating body 140 of the first bridging valve 29 and return valve 28 is placed between the return conduit 24 and the bridging conduit 25, the common rotating body 140 of the first withdrawal valve 26 and second bridging valve 30 is placed between the withdrawal conduit 23 and the bridging conduit 25, the common rotating body 140 of the access valve 32 and withdrawal valve 27 is placed between the withdrawal conduit 23 and the pre-blood pump infusion line 18', the common rotating body 140 of the second infusion valve 110 and auxiliary withdrawal valve 126 is placed between the withdrawal conduit 23 and the pre-blood pump infusion line 18'.

The position of the valves 26, 27, 28, 29, 30, 32, 110, 126 configured to perform patient treatment is shown in Figure 16. The first bridging valve 29, the second bridging valve 30, the access valve 32 and the second infusion valve 110 are closed, the first withdrawal valve 26, the second withdrawal valve 27, the auxiliary withdrawal valve 126 and the return valve 28 are open.

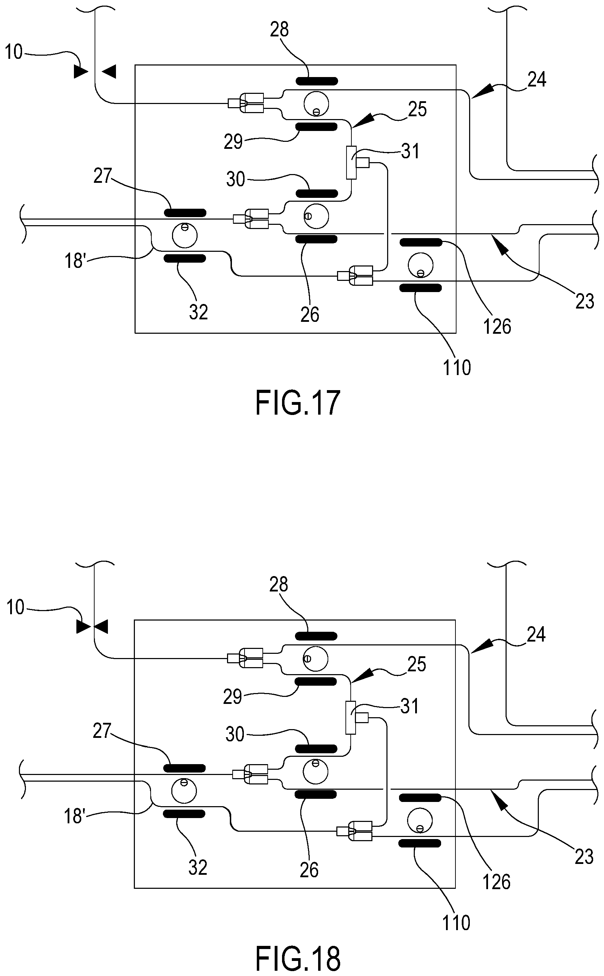

The position of the valves 26, 27, 28, 29, 30, 32, 110, 126 configured to perform flushing of the withdrawal conduit 23 and of the vascular access of the patient "P" is shown in Figure 17. The first bridging valve 29, the second withdrawal valve 27 and the second infusion valve 110 are closed while the access valve 32, the second bridging valve 30, the auxiliary withdrawal valve 126 and the first withdrawal valve 26 are open. Fluid from the pre-blood pump infusion fluid container 19' flows through the access point 31 into the withdrawal conduit 23 and towards to patient "P".

The position of the valves 26, 27, 28, 29, 30, 32, 110, 126 configured to perform flushing of the return conduit 24 is shown in Figure 18. The first bridging valve 29, the return valve 28, the access valve 32, the first withdrawal valve 26 and the auxiliary withdrawal valve 126 are open while the second withdrawal valve 27, the second bridging valve 30 and the second infusion valve 110 are closed. Fluid from the pre-blood pump infusion fluid container 19' flows through the access point 31 into the return conduit 24 and towards to patient "P".

The position of the valves 26, 27, 28, 29, 30, 32, 110, 126 configured to perform blood recirculation in the blood treatment unit 2 of the apparatus 1 is shown in Figure 19.

The return valve 28, the first withdrawal valve 26, the second infusion valve 110 and the access valve 32 are closed while the second withdrawal valve 27, the first and second bridging valves 29, 30 and the auxiliary withdrawal valve 126 are open.

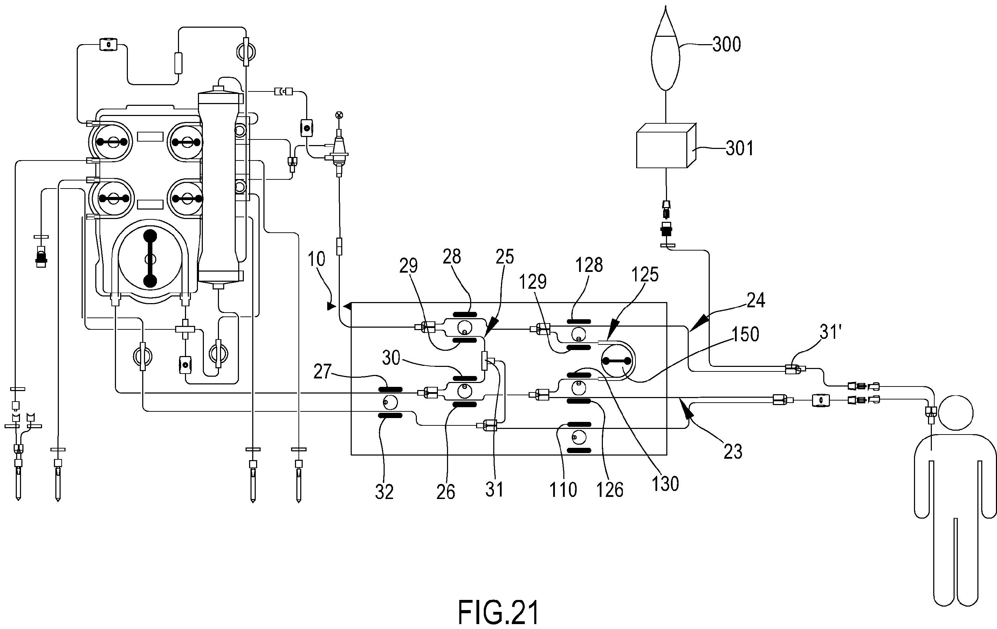

Figures 21 to 26 show a variant of the embodiment Figures 16 to 20B. With respect to Figures 16 to 20B, this variant additionally comprises an auxiliary bridging conduit 125 which is arranged in parallel with respect to the bridging conduit 25 and also connects or is configured to connect the withdrawal conduit 23 to the return conduit 24. The auxiliary bridging conduit 125 is positioned between the bridging conduit 25 and the access device 8. With respect to a flow of blood during treatment, the auxiliary bridging conduit 125 is connected to the withdrawal conduit 23 at a connection site placed upstream the bridging conduit 25 and is connected to the return conduit 6 at a connection site placed downstream the bridging conduit 25.

An auxiliary pump 150, e.g. a peristaltic pump, operates on the auxiliary bridging conduit 125 and is configured to recirculate blood on a patient "P" side.

The auxiliary withdrawal valve 126 operates on the withdrawal conduit 23 like in Figures 16 to 20B and an auxiliary return valve 128 operates on the return conduit 24. The auxiliary return valve 128 is placed downstream, with respect to a flow of blood during treatment, the return valve 28. With respect to said flow of blood during treatment, the auxiliary withdrawal valve 126 is placed upstream the connection site of the auxiliary bridging conduit 125 to the withdrawal conduit 23 and the auxiliary return valve 128 is placed downstream the connection site of the auxiliary bridging conduit 125 to the return conduit 24.

A first auxiliary bridging valve 129 and a second auxiliary bridging valve 130 operate on the auxiliary bridging conduit 125 and are placed on opposite sides with respect to the auxiliary pump 150.

The first auxiliary bridging valve 129 and the auxiliary return valve 128 comprise one common rotating body 140 and two respective abutment elements 145. Likewise, the auxiliary withdrawal valve 126 and the second auxiliary bridging valve 130 comprise one common rotating body 140 and two respective abutment elements 145. As shown in Figures 21 to 25, the common rotating body 140 of the first auxiliary bridging valve 129 and auxiliary return valve 128 is placed between the return conduit 24 and the auxiliary bridging conduit 125. The common rotating body 140 of the auxiliary withdrawal valve 126 and the second auxiliary bridging valve 130 is placed between the withdrawal conduit 23 and the auxiliary bridging conduit 125.

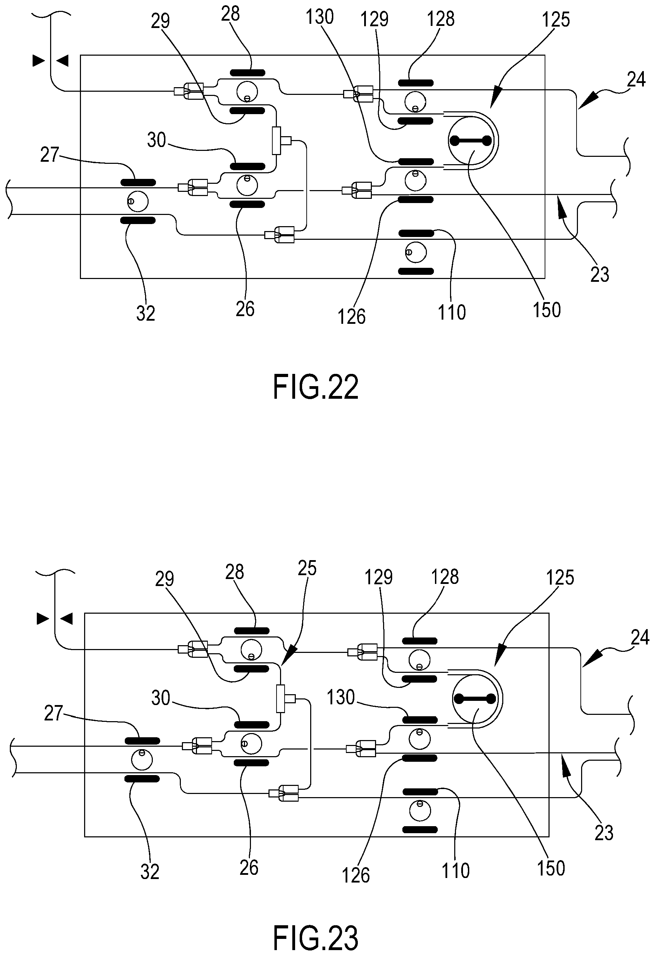

During patient treatment (Figures 21 and 22), the first auxiliary bridging valve 129 and the second auxiliary bridging valve 130 are closed while the auxiliary withdrawal valve 126 and the auxiliary return valve 128 are open. The position of the other valves may the same as shown in Figure 16. The access valve 32 may be open and the pre-blood pump infusion line 18' works to supply replacement fluid from the pre-blood pump infusion fluid container 19' to the blood withdrawal line 6.

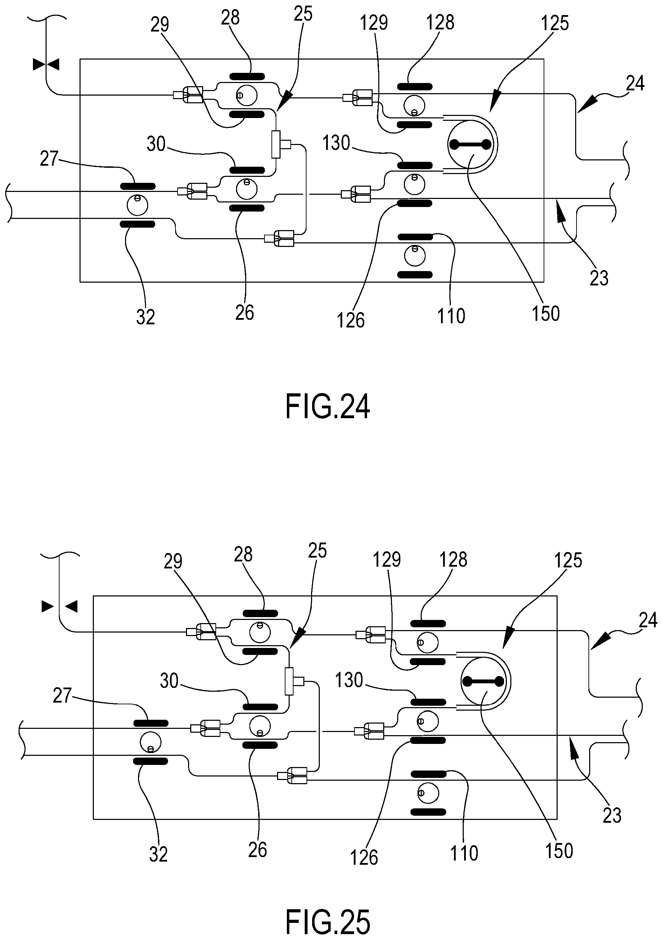

When flushing the withdrawal conduit 23 (Figure 23) or the return conduit 24 (Figure 24), the auxiliary withdrawal valve 126 and the auxiliary return valve 128 are open while the first auxiliary bridging valve 129 and the second auxiliary bridging valve 130 are closed. The position of the other valves may the same as shown respectively in Figure 17 or Figure 18.

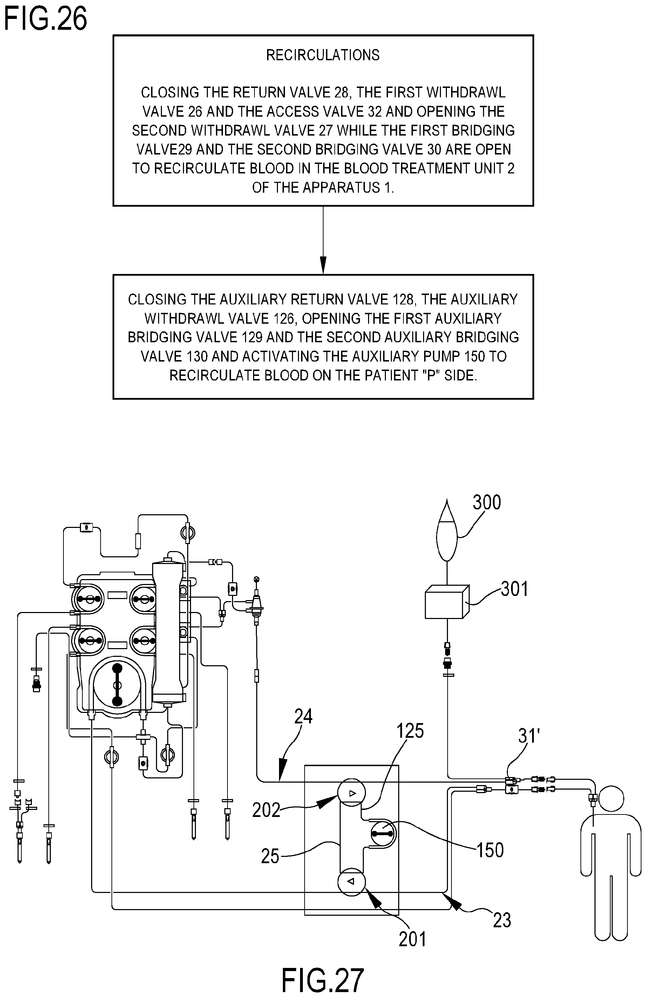

During blood recirculation (Figures 25 and 26), the auxiliary withdrawal valve 126, the auxiliary return valve 128, the first auxiliary bridging valve 126 and the second auxiliary bridging valve all are open. The position of the other valves may the same as shown in Figure 19. The blood pump 11 works to recirculate blood through the treatment unit 2 and the auxiliary pump 150 is activated to recirculate blood on the patient "P" side.

Figures 27 to 30 show another different embodiment of the fluid diverting device 21. The fluid diverting device 21 of this embodiment comprises distributors 201, 202 in place of valves. The distributors 201, 202 may be disposable. The distributors 201, 202 may be rotary distributors and may be operated through rotary actuators, not shown, part of the main portion 22 and controlled by the control unit 100.

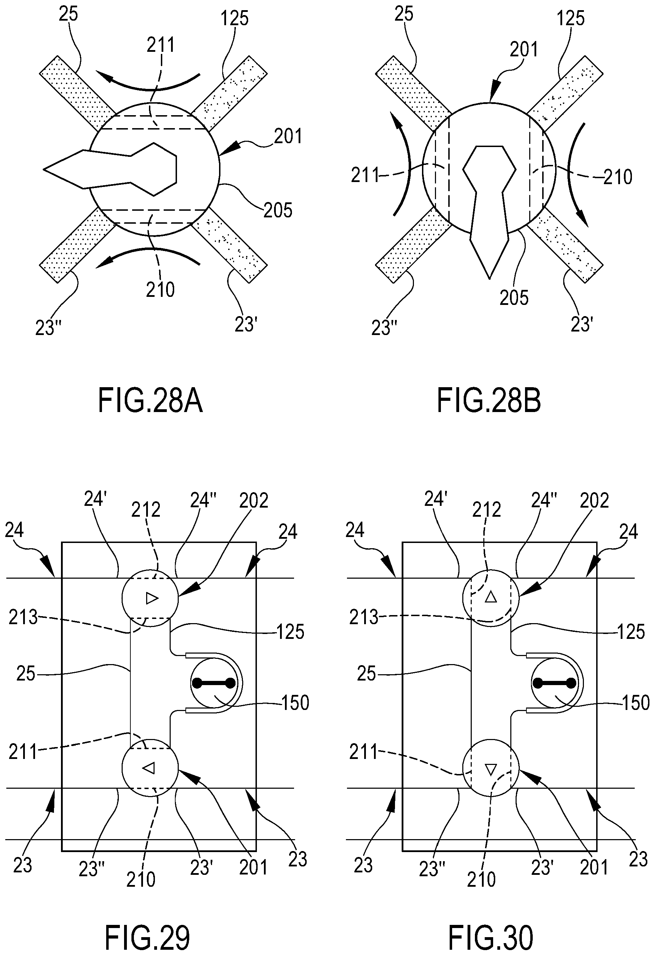

The fluid diverting device 21 comprises (Figures 28 and 29) the withdrawal conduit 23, the return conduit 24, the bridging conduit 25 and the auxiliary bridging conduit 125. The withdrawal conduit 23 comprises a first branch 23' and a second branch 23'' . Likewise, the return conduit 24 comprises a first branch 24' and a second branch 24''. The auxiliary bridging conduit 125 is coupled to the auxiliary pump 150.

A first distributor 201 is operationally coupled to the withdrawal conduit 23, to the bridging conduit 25 and to the auxiliary bridging conduit 125. The first distributor 201 is operative between the first branch 23' and the second branch 23'' of the withdrawal conduit 23. The first distributor 201 is also operative between a first end of the bridging conduit 25 and a first end of the auxiliary bridging conduit 125.

A second distributor 202 is operationally coupled to the return conduit 24, to the bridging conduit 25 and to the auxiliary bridging conduit 125. The second distributor 202 is operative between the first branch 24' and the second branch 24'' of the return conduit 24. The second distributor 202 is also operative between a second end of the bridging conduit 25 and a second end of the auxiliary bridging conduit 125.

As better shown in Figures 28A and 28B, the first distributor 201 comprises a rotating element 205 provided with a first through passage 210 and a second through passage 211. The first through passage 210 and the second through passage 211 are parallel and each has open ends facing on opposite parts of the rotating element 205. The second distributor 202 comprises a rotating element 205 provided with a first through passage 212 and a second through passage 213. The structure of the second distributor 202 (not shown in an enlarged view) is the same as the first distributor 201.

Each of the first distributor 201 and the second distributor 202 may be rotated between: a first configuration corresponding to the treatment configuration and a second configuration corresponding to the recirculation configuration.

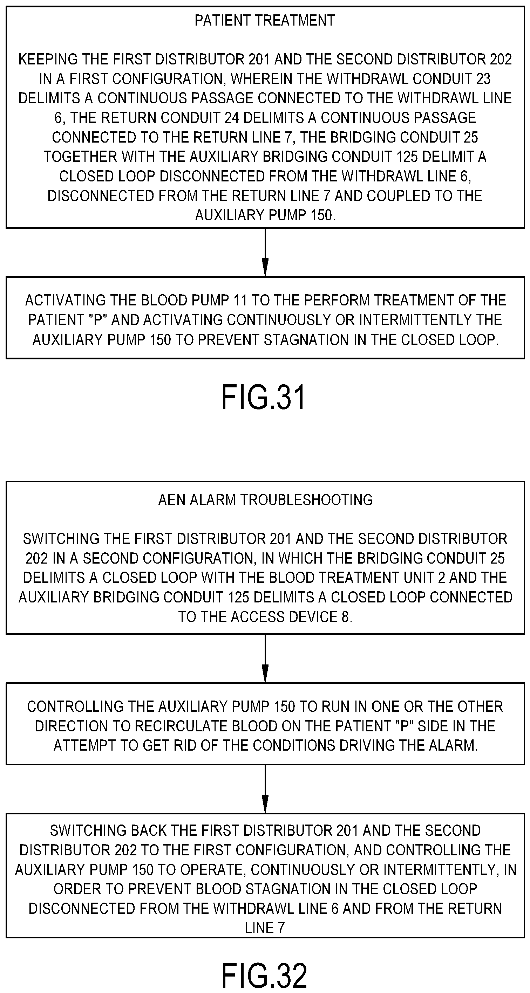

In the first configuration of Figures 27, 28A, 29 and 31, the withdrawal conduit 23 delimits a continuous passage connected to the withdrawal line 6.

The first through passage 210 of the first distributor 201 connects the first branch 23' and the second branch 23'' of the withdrawal conduit 23 and the second through passage 211 of the first distributor 201 connects the first end of the bridging conduit 25 to the first end of the auxiliary bridging conduit 125. In this first configuration, the return conduit 24 delimits a continuous passage connected to the return line 7 (Figure 29).

The first through passage 212 of the second distributor 202 connects a first branch 24' and a second branch 24'' of the return conduit 24 and the second through passage 213 of the second distributor 202 connects the second end of the bridging conduit 25 to the second end of the auxiliary bridging conduit 125.

In this first configuration, the bridging conduit 25 together with the auxiliary bridging conduit 125 delimit a closed loop disconnected from the withdrawal line 6, disconnected from the return line 7 and coupled to the auxiliary pump 150.

In the second configuration of Figures 28B, 30 and 32, the bridging conduit 25 delimits a closed loop with the blood treatment unit 2 to recirculate blood in the blood treatment unit 2 and the auxiliary bridging conduit 125 delimits a closed loop connected to the access device 8, to recirculate blood on the patient "P" side. The closed loop on the side of the blood treatment unit 2 is disconnected from the closed loop on the patient "P" side.

In this second configuration, the first through passage 210 of the first distributor 201 connects the first branch 23' of the withdrawal conduit 23 to the auxiliary bridging conduit 125 and the second through passage 211 of the first distributor 201 connects the second branch 23'' of the withdrawal conduit 23 to the bridging conduit 25 (Figures 28B and 30).

In this second configuration, the first through passage 212 of the second distributor 202 connects the first branch 24' of the return conduit 24 to the bridging conduit 25 and the second through passage 213 of the second distributor 202 connects the second branch 24'' of the return conduit 24 to the auxiliary bridging conduit 125 (Figure 30).

In case of Access Extremely Negative (AEN) alarm during blood treatment, the control unit 100 switches the first and second distributors 201, 202 from the first configuration to the second configuration. The auxiliary pump 150 is controlled to run in one or the other direction in the attempt to get rid of the conditions driving the alarm. Then the distributors 201, 202 would switch back to the first configuration (treatment mode), and the auxiliary pump 150 would operate at low rate, continuously or intermittently, in order to prevent blood stagnation.

## Claims

1. Fluid diverting device for an apparatus for extracorporeal treatment of blood, wherein the apparatus (1) comprises:
a blood treatment unit (2);
an extracorporeal blood circuit coupled to the blood treatment unit (2) and comprising a blood withdrawal line (6) and a blood return line (7) connectable to a vascular access of a patient (P);
a blood pump (11) configured to be coupled to a pump section
of the extracorporeal blood circuit;
wherein the fluid diverting device (21) is configured to be placed in-line between a blood set of the apparatus (1) and the vascular access of the patient (P) and comprises:
- a substantially H-shaped conduits assembly comprising a withdrawal conduit (23), a return conduit (24) and at least one bridging conduit (25, 125) connecting or configured to connect the withdrawal conduit (23) to the return conduit (24); wherein the withdrawal conduit (23) is connected or connectable upstream and downstream to the withdrawal line (6), wherein the return conduit (24) is connected or connectable upstream and downstream to the return line (7);
- a plurality of valves (26, 27, 28, 29, 30, 32) or distributors (201, 202) operating on the withdrawal conduit (23), on the return conduit (24) and on the at least one bridging conduit (25) and configured to divert a flow of liquid and/or blood without disconnecting the patient (P);
**characterized in that** at least one of the plurality of valves (26, 27, 28, 29, 30, 32) is a rotating clamp, wherein the rotating clamp comprises a rotating body (140) and an abutment element (145) ;
and **in that** at least two valves of the plurality of valves (26, 27, 28, 29, 30, 32) comprise one common rotating body (140) and respective abutment elements (145).

2. The device according to claim 1, wherein the withdrawal conduit (23) is substantially parallel to the return conduit (24) and wherein the bridging conduit (25) is at least in part transversal, optionally perpendicular, to the withdrawal conduit (23) and to the return conduit (24).

3. The device according to claim 1 or 2, wherein at least one of the withdrawal conduit (23), the return conduit (24) and the bridging conduit (25) is at least in part straight.

4. The device according to any of claims 1 to 3, wherein the plurality of valves (26, 27, 28, 29, 30, 32) comprises at least a withdrawal valve (26, 27) operating on the withdrawal conduit (23), at least a return valve (28) operating on the return conduit (24) and at least a bridging valve (29, 30) operating on the bridging conduit (25).

5. The device according to claim 4, wherein said at least a withdrawal valve (26, 27) comprises a first withdrawal valve (26) and a second withdrawal valve (27) operating on the withdrawal conduit (23), wherein the first withdrawal valve (26) and the second withdrawal valve (27) are placed on opposite sides with respect to a junction of the bridging conduit (25) to the withdrawal conduit (23), wherein the first withdrawal valve (26) is placed upstream, with respect to a flow of blood during treatment, the junction of the bridging conduit (25) to said withdrawal conduit (23), wherein the second withdrawal valve (27) is placed downstream, with respect to the flow of blood during treatment, the junction of the bridging conduit (25) to said withdrawal conduit (23).

6. The device according to claim 4 or 5, wherein the return valve (28) is placed downstream a junction of the bridging conduit (25) to the return conduit (24) with respect to a flow of blood during treatment.

7. The device according to claim 4 or 5 or 6, wherein said at least a bridging valve (29, 30) comprises a first bridging valve (29) and a second bridging valve (30) operating on the bridging conduit (25), wherein the first bridging valve (29) is placed closer to the return conduit (24) and the second bridging valve (30) is placed closer to the withdrawal conduit (23); optionally, wherein the bridging conduit (25) has an access point (31) located between the first bridging valve (29) and the second bridging valve (30) and suitable to connect a liquid source (33; 19'), optionally a saline source, optionally a syringe or a bag, or optionally a citrate source coming from the apparatus (1) for extracorporeal blood treatment.

8. The device according to claim 7, comprising a liquid source, optionally a syringe or a bag, connected or connectable to the access point (31).

9. The device according to any of claims 1 to 8, comprising a holder (34) configured to hold the substantially H-shaped conduits assembly and the plurality of valves (26, 27, 28, 29, 30, 32) and, optionally when claim 9 depends on claim 8, the liquid source (33; 19').

10. The device according to any of claims 4 to 8, wherein the plurality of valves (26, 27, 28, 29, 30, 32) is configured to be arranged at least in a treatment configuration, in which said at least a withdrawal valve (26, 27) and said at least a return valve (28) are open and said at least a bridging valve (29, 30) is closed, to perform treatment of the patient (P).

11. The device according to claim 6 when depending on claim 5, wherein the plurality of valves (26, 27, 28, 29, 30, 32) is configured to be arranged at least in a recirculation configuration, in which the return valve (28) is closed, the first withdrawal valve (26) is closed, the second withdrawal valve (27) is open and said at least a bridging valve (29, 30) is open, so that blood or liquid recirculates in the blood treatment unit (2) of the apparatus (1) without disconnecting the patient (P).

12. The device according to claim 7 when dependent on claim 5, wherein the plurality of valves (26, 27, 28, 29, 30, 32) is configured to be arranged in a return branch flushing configuration, in which the first bridging valve (29) is open, the second bridging valve (30) is closed and the liquid source (33; 19') is connected to the access point (31) to inject liquid and flush a branch of the return conduit (24) placed downstream a junction of the bridging conduit (25) to the return conduit (24).

13. The device according to claim 12, wherein the plurality of valves (26, 27, 28, 29, 30, 32) is configured to be arranged in a withdrawal branch flushing configuration, in which the first bridging valve (29) is closed, the second bridging valve (30) is open, the second withdrawal valve (27) is closed and the liquid source (33; 19') is connected to the access point (31) to inject liquid and flush a branch of the withdrawal conduit (23) placed upstream a junction of the bridging conduit (25) to the withdrawal conduit (23).

14. The device according to any of claims 10 to 13, wherein the valves of the plurality of valves (26, 27, 28, 29, 30, 32) are operatively connected or connectable to a control unit (100), optionally of the apparatus (1) for extracorporeal treatment of blood, or wherein the fluid diverting device (21) comprises a control unit operatively connected to the valves of the plurality of valves (26, 27, 28, 29, 30, 32); wherein the control unit (100) is configured for commanding, optionally automatically, the configurations of the plurality of valves (26, 27, 28, 29, 30, 32) .

15. A blood set for an apparatus for extracorporeal treatment of blood, the blood set comprising:
a blood treatment unit (2);
an extracorporeal blood circuit coupled to the blood treatment unit (2) and comprising a blood withdrawal line (6) and a blood return line (7) connectable to a vascular access of a patient (P),
optionally each of the blood withdrawal line (6) and the blood return line (7) having a respective end connector configured to be connected to the vascular access of the patient (P);
a fluid diverting device (21) according to one of the preceding claims 1 to 14, wherein the fluid diverting device(21) is placed or is configured to be placed in-line between the blood set of the apparatus (1) for extracorporeal treatment of blood and the vascular access of the patient (P), in particular the withdrawal conduit (23) and the return conduit (24) having a respective end counter-connector configured to be coupled or coupled with the end connector of the blood withdrawal line (6) and of the blood return line (7).

## Patentansprüche

1. Fluidumlenkvorrichtung für ein Gerät für die extrakorporale Behandlung von Blut, wobei das Gerät (1) Folgendes umfasst:
eine Blutbehandlungseinheit (2),
einen extrakorporalen Blutkreislauf, der an die Blutbehandlungseinheit (2) gekoppelt ist und eine Blutentnahmeleitung (6) und eine Blutrückgabeleitung (7) umfasst, die mit einem Gefäßzugang eines Patienten (P) verbindbar sind,
eine Blutpumpe (11), die zum Koppeln an einen Pumpenabschnitt des extrakorporalen Blutkreislaufs ausgestaltet ist,
wobei die Fluidumlenkvorrichtung (21) dazu ausgestaltet ist, in-line zwischen einem Blutsatz des Geräts (1) und dem Gefäßzugang des Patienten (P) platziert zu werden, und Folgendes umfasst:
- eine im Wesentlichen H-förmige Kanalbaugruppe, die einen Entnahmekanal (23), einen Rückgabekanal (24) und mindestens einen Überbrückungskanal (25, 125) umfasst, der den Entnahmekanal (23) mit dem Rückgabekanal (24) verbindet oder dazu ausgestaltet ist, diese zu verbinden, wobei der Entnahmekanal (23) stromaufwärtig und stromabwärtig mit der Entnahmeleitung (6) verbunden oder verbindbar ist, wobei der Rückgabekanal (24) stromaufwärtig und stromabwärtig mit der Rückgabeleitung (7) verbunden oder verbindbar ist,
- eine Vielzahl von Ventilen (26, 27, 28, 29, 30, 32) oder Verteilern (201, 202), die an dem Entnahmekanal (23), an dem Rückgabekanal (24) und an dem mindestens einen Überbrückungskanal (25) wirken und dazu ausgestaltet sind, einen Flüssigkeits- und/oder Blutstrom ohne Abtrennung von dem Patienten (P) umzulenken,
**dadurch gekennzeichnet, dass** mindestens eines der Vielzahl von Ventilen (26, 27, 28, 29, 30, 32) eine Drehklemme ist, wobei die Drehklemme einen Drehkörper (140) und ein Anschlagelement (145) umfasst,
und dass mindestens zwei Ventile der Vielzahl von Ventilen (26, 27, 28, 29, 30, 32) einen gemeinsamen Drehkörper (140) und jeweilige Anschlagelemente (145) umfassen.

2. Vorrichtung nach Anspruch 1, wobei der Entnahmekanal (23) im Wesentlichen parallel zu dem Rückgabekanal (24) verläuft und wobei der Überbrückungskanal (25) mindestens zum Teil quer, optional senkrecht, zu dem Entnahmekanal (23) und dem Rückgabekanal (24) verläuft.

3. Vorrichtung nach Anspruch 1 oder 2, wobei der Entnahmekanal (23) und/oder der Rückgabekanal (24) und/oder der Überbrückungskanal (25) mindestens zum Teil gerade sind.

4. Vorrichtung nach einem der Ansprüche 1 bis 3, wobei die Vielzahl von Ventilen (26, 27, 28, 29, 30, 32) mindestens ein Entnahmeventil (26, 27), das an dem Entnahmekanal (23) wirkt, mindestens ein Rückgabeventil (28), das an dem Rückgabekanal (24) wirkt, und mindestens ein Überbrückungsventil (29, 30), das an dem Überbrückungskanal (25) wirkt, umfassen.

5. Vorrichtung nach Anspruch 4, wobei das mindestens eine Entnahmeventil (26, 27) ein erstes Entnahmeventil (26) und ein zweites Entnahmeventil (27) umfasst, die an dem Entnahmekanal (23) wirken, wobei das erste Entnahmeventil (26) und das zweite Entnahmeventil (27) an gegenüberliegenden Seiten bezüglich einer Verbindungsstelle des Überbrückungskanals (25) mit dem Entnahmekanal (23) platziert sind, wobei das erste Entnahmeventil (26) bezüglich einer Blutströmung während der Behandlung stromaufwärts von der Verbindungsstelle des Überbrückungskanals (25) mit dem Entnahmekanal (23) platziert ist, wobei das zweite Entnahmeventil (27) bezüglich der Blutströmung während der Behandlung stromabwärts von der Verbindungsstelle des Überbrückungskanals (25) mit dem Entnahmekanal (23) platziert ist.

6. Vorrichtung nach Anspruch 4 oder 5, wobei das Rückgabeventil (28) bezüglich einer Blutströmung während der Behandlung stromabwärts von einer Verbindungsstelle des Überbrückungskanals (25) mit dem Rückgabekanal (24) platziert ist.

7. Vorrichtung nach Anspruch 4 oder 5 oder 6, wobei das mindestens eine Überbrückungsventil (29, 30) ein erstes Überbrückungsventil (29) und ein zweites Überbrückungsventil (30) umfasst, die an dem Überbrückungskanal (25) wirken, wobei das erste Überbrückungsventil (29) näher an dem Rückgabekanal (24) platziert ist und das zweite Überbrückungsventil (30) näher an dem Entnahmekanal (23) platziert ist, optional wobei der Überbrückungskanal (25) einen Zugangspunkt (31) hat, der zwischen dem ersten Überbrückungsventil (29) und dem zweiten Überbrückungsventil (30) angeordnet und zum Anschluss einer Flüssigkeitsquelle (33; 19'), optional einer Kochsalzlösungsquelle, optional einer Spritze oder eines Beutels, oder optional einer Citratquelle, die von dem Gerät (1) für die extrakorporale Blutbehandlung kommt, geeignet ist.

8. Vorrichtung nach Anspruch 7, umfassend eine Flüssigkeitsquelle, optional eine Spritze oder einen Beutel, die bzw. der mit dem Zugangspunkt (31) verbunden oder verbindbar ist.

9. Vorrichtung nach einem der Ansprüche 1 bis 8, umfassend einen Halter (34), der zum Halten der im Wesentlichen H-förmigen Kanalbaugruppe und der Vielzahl von Ventilen (26, 27, 28, 29, 30, 32) und, optional wenn Anspruch 9 von Anspruch 8 abhängig ist, der Flüssigkeitsquelle (33; 19') ausgestaltet ist.

10. Vorrichtung nach einem der Ansprüche 4 bis 8, wobei die Vielzahl von Ventilen (26, 27, 28, 29, 30, 32) zur Anordnung mindestens in einer Behandlungskonfiguration ausgestaltet sind, in der das mindestens eine Entnahmeventil (26, 27) und das mindestens eine Rückgabeventil (28) offen sind und das mindestens eine Überbrückungsventil (29, 30) geschlossen ist, um die Behandlung des Patienten (P) durchzuführen.

11. Vorrichtung nach Anspruch 6, wenn von Anspruch 5 abhängig, wobei die Vielzahl von Ventilen (26, 27, 28, 29, 30, 32) zur Anordnung mindestens in einer Rezirkulationskonfiguration ausgestaltet sind, in der das Rückgabeventil (28) geschlossen ist, das erste Entnahmeventil (26) geschlossen ist, das zweite Entnahmeventil (27) offen ist und das mindestens eine Überbrückungsventil (29, 30) offen ist, so dass Blut oder Flüssigkeit in der Blutbehandlungseinheit (2) des Geräts (1) rezirkuliert, ohne Abtrennung von dem Patienten (P).

12. Vorrichtung nach Anspruch 7, wenn von Anspruch 5 abhängig, wobei die Vielzahl von Ventilen (26, 27, 28, 29, 30, 32) zur Anordnung in einer Rückgabezweig-Spülkonfiguration ausgestaltet sind, in der das erste Überbrückungsventil (29) offen ist, das zweite Überbrückungsventil (30) geschlossen ist und die Flüssigkeitsquelle (33; 19') mit dem Zugangspunkt (31) verbunden ist, um Flüssigkeit zu injizieren und einen Zweig des Rückgabekanals (24), der stromabwärts einer Verbindungsstelle des Überbrückungskanals (25) mit dem Rückgabekanal (24) platziert ist, zu spülen.

13. Vorrichtung nach Anspruch 12, wobei die Vielzahl von Ventilen (26, 27, 28, 29, 30, 32) zur Anordnung in einer Entnahmezweig-Spülkonfiguration ausgestaltet sind, in der das erste Überbrückungsventil (29) geschlossen ist, das zweite Überbrückungsventil (30) offen ist, das zweite Entnahmeventil (27) geschlossen ist und die Flüssigkeitsquelle (33; 19') mit dem Zugangspunkt (31) verbunden ist, um Flüssigkeit zu injizieren und einen Zweig des Entnahmekanals (23), der stromaufwärts einer Verbindungsstelle des Überbrückungskanals (25) mit dem Entnahmekanal (23) platziert ist, zu spülen.

14. Vorrichtung nach einem der Ansprüche 10 bis 13, wobei die Vielzahl von Ventilen (26, 27, 28, 29, 30, 32) mit einer Steuereinheit (100), optional des Geräts (1) für die extrakorporale Behandlung von Blut, wirkverbunden oder wirkverbindbar sind oder wobei die Fluidumlenkvorrichtung (21) eine Steuereinheit umfasst, die mit der Vielzahl von Ventilen (26, 27, 28, 29, 30, 32) wirkverbunden ist, wobei die Steuereinheit (100) zu der, optional automatischen, Steuerung der Konfigurationen der Vielzahl von Ventilen (26, 27, 28, 29, 30, 32) ausgestaltet ist.

15. Blutsatz für ein Gerät für die extrakorporale Behandlung von Blut, wobei der Blutsatz Folgendes umfasst:
eine Blutbehandlungseinheit (2),
einen extrakorporalen Blutkreislauf, der an die Blutbehandlungseinheit (2) gekoppelt ist und eine Blutentnahmeleitung (6) und eine Blutrückgabeleitung (7) umfasst, die mit einem Gefäßzugang eines Patienten (P) verbindbar sind, wobei optional die Blutentnahmeleitung (6) und die Blutrückgabeleitung (7) jeweils einen jeweiligen Endverbinder haben, der zur Verbindung mit dem Gefäßzugang des Patienten (P) ausgestaltet ist,
eine Fluidumlenkvorrichtung (21) nach einem der vorhergehenden Ansprüche 1 bis 14, wobei die Fluidumlenkvorrichtung (21) in-line zwischen dem Blutsatz des Geräts (1) für die extrakorporale Behandlung von Blut und dem Gefäßzugang des Patienten (P) platziert oder zum derartigen Platzieren ausgestaltet ist, wobei insbesondere der Entnahmekanal (23) und der Rückgabekanal (24) einen jeweiligen Endgegenverbinder haben, der zum Koppeln mit dem Endverbinder der Blutentnahmeleitung (6) und der Blutrückgabeleitung (7) ausgestaltet oder mit diesen gekoppelt ist.

## Revendications

1. Dispositif de déviation de fluide pour un appareil de traitement extracorporel du sang, l'appareil (1) comprenant :
une unité de traitement du sang (2) ;
un circuit sanguin extracorporel accouplé à l'unité de traitement du sang (2) et comprenant une ligne de prélèvement de sang (6) et une ligne de retour de sang (7) pouvant être raccordées à un accès vasculaire d'un patient (P) ;
une pompe à sang (11) conçue pour être accouplée à une section de pompe du circuit sanguin extracorporel ;
le dispositif de déviation de fluide (21) étant conçu pour être placé en ligne entre un ensemble pour le sang de l'appareil (1) et l'accès vasculaire du patient (P) et comprenant :
- un ensemble de conduits sensiblement en forme de H comprenant un conduit de prélèvement (23), un conduit de retour (24) et au moins un conduit de liaison (25, 125) raccordant ou conçu pour raccorder le conduit de prélèvement (23) au conduit de retour (24) ; le conduit de prélèvement (23) étant raccordé ou pouvant être raccordé en amont et en aval à la ligne de prélèvement (6), le conduit de retour (24) étant raccordé ou pouvant être raccordé en amont et en aval à la ligne de retour (7) ;
- une pluralité de vannes (26, 27, 28, 29, 30, 32) ou de distributeurs (201, 202) fonctionnant sur le conduit de prélèvement (23), sur le conduit de retour (24) et sur l'au moins un conduit de liaison (25) et conçue pour dévier un flux de liquide et/ou de sang sans débrancher le patient (P) ;
**caractérisé en ce qu'**au moins une vanne de la pluralité de vannes (26, 27, 28, 29, 30, 32) est une pince rotative, la pince rotative comprenant un corps rotatif (140) et un élément de butée (145) ;
et **en ce qu'**au moins deux vannes de la pluralité de vannes (26, 27, 28, 29, 30, 32) comprennent un corps rotatif commun (140) et des éléments de butée (145) respectifs.

2. Dispositif selon la revendication 1, le conduit de prélèvement (23) étant sensiblement parallèle au conduit de retour (24) et le conduit de liaison (25) étant au moins en partie transversal, éventuellement perpendiculaire, au conduit de prélèvement (23) et au conduit de retour (24).

3. Dispositif selon la revendication 1 ou 2, au moins l'un parmi le conduit de prélèvement (23), le conduit de retour (24) et le conduit de liaison (25) étant au moins en partie droit.

4. Dispositif selon l'une quelconque des revendications 1 à 3, la pluralité de vannes (26, 27, 28, 29, 30, 32) comprenant au moins une vanne de prélèvement (26, 27) fonctionnant sur le conduit de prélèvement (23), au moins une vanne de retour (28) fonctionnant sur le conduit de retour (24) et au moins une vanne de liaison (29, 30) fonctionnant sur le conduit de liaison (25).

5. Dispositif selon la revendication 4, ladite au moins une vanne de prélèvement (26, 27) comprenant une première vanne de prélèvement (26) et une deuxième vanne de prélèvement (27) fonctionnant sur le conduit de prélèvement (23), la première vanne de prélèvement (26) et la deuxième vanne de prélèvement (27) étant placées sur des côtés opposés par rapport à une jonction du conduit de liaison (25) avec le conduit de prélèvement (23), la première vanne de prélèvement (26) étant placée en amont, par rapport à un flux de sang pendant le traitement, de la jonction du conduit de liaison (25) avec ledit conduit de prélèvement (23), la deuxième vanne de prélèvement (27) étant placée en aval, par rapport au flux de sang pendant le traitement, de la jonction du conduit de liaison (25) avec ledit conduit de prélèvement (23) .

6. Dispositif selon la revendication 4 ou 5, la vanne de retour (28) étant placée en aval d'une jonction du conduit de liaison (25) avec le conduit de retour (24) par rapport à un flux de sang pendant le traitement.

7. Dispositif selon la revendication 4 ou 5 ou 6, ladite au moins une vanne de liaison (29, 30) comprenant une première vanne de liaison (29) et une deuxième vanne de liaison (30) fonctionnant sur le conduit de liaison (25), la première vanne de liaison (29) étant placée plus près du conduit de retour (24) et la deuxième vanne de liaison (30) étant placée plus près du conduit de prélèvement (23) ; éventuellement le conduit de liaison (25) ayant un point d'accès (31) situé entre la première vanne de liaison (29) et la deuxième vanne de liaison (30) et approprié pour raccorder une source de liquide (33 ; 19'), éventuellement une source de solution saline, éventuellement une seringue ou une poche, ou éventuellement une source de citrate venant de l'appareil (1) de traitement extracorporel du sang.

8. Dispositif selon la revendication 7, comprenant une source de liquide, éventuellement une seringue ou une poche, raccordée ou pouvant être raccordée au point d'accès (31).

9. Dispositif selon l'une quelconque des revendications 1 à 8, comprenant un support (34) conçu pour supporter l'ensemble de conduits sensiblement en forme de H et la pluralité de vannes (26, 27, 28, 29, 30, 32) et, éventuellement lorsque la revendication 9 dépend de la revendication 8, la source de liquide (33 ; 19').

10. Dispositif selon l'une quelconque des revendications 4 à 8, la pluralité de vannes (26, 27, 28, 29, 30, 32) étant conçue pour être agencée au moins dans une configuration de traitement, dans laquelle ladite au moins une vanne de prélèvement (26, 27) et ladite au moins une vanne de retour (28) sont ouvertes et ladite au moins une vanne de liaison (29, 30) est fermée, pour réaliser le traitement du patient (P).

11. Dispositif selon la revendication 6 lorsqu'elle dépend de la revendication 5, la pluralité de vannes (26, 27, 28, 29, 30, 32) étant conçue pour être agencée au moins dans une configuration de recirculation, dans laquelle la vanne de retour (28) est fermée, la première vanne de prélèvement (26) est fermée, la deuxième vanne de prélèvement (27) est ouverte et ladite au moins une vanne de liaison (29, 30) est ouverte, de sorte que le sang ou le liquide recircule dans l'unité de traitement du sang (2) de l'appareil (1) sans débrancher le patient (P) .

12. Dispositif selon la revendication 7 lorsqu'elle dépend de la revendication 5, la pluralité de vannes (26, 27, 28, 29, 30, 32) étant conçue pour être agencée dans une configuration de rinçage de branche de retour, dans laquelle la première vanne de liaison (29) est ouverte, la deuxième vanne de liaison (30) est fermée et la source de liquide (33 ; 19') est raccordée au point d'accès (31) pour injecter un liquide et rincer une branche du conduit de retour (24) placée en aval d'une jonction du conduit de liaison (25) avec le conduit de retour (24).

13. Dispositif selon la revendication 12, la pluralité de vannes (26, 27, 28, 29, 30, 32) étant conçue pour être agencée dans une configuration de rinçage de branche de prélèvement, dans laquelle la première vanne de liaison (29) est fermée, la deuxième vanne de liaison (30) est ouverte, la deuxième vanne de prélèvement (27) est fermée et la source de liquide (33 ; 19') est raccordée au point d'accès (31) pour injecter un liquide et rincer une branche du conduit de prélèvement (23) placée en amont d'une jonction du conduit de liaison (25) avec le conduit de prélèvement (23).

14. Dispositif selon l'une quelconque des revendications 10 à 13, les vannes de la pluralité de vannes (26, 27, 28, 29, 30, 32) étant raccordées ou pouvant être raccordées de manière opérationnelle à une unité de commande (100), éventuellement de l'appareil (1) de traitement extracorporel du sang, ou le dispositif de déviation de fluide (21) comprenant une unité de commande raccordée de manière opérationnelle aux vannes de la pluralité de vannes (26, 27, 28, 29, 30, 32) ; l'unité de commande (100) étant conçue pour commander, éventuellement automatiquement, les configurations de la pluralité de vannes (26, 27, 28, 29, 30, 32).

15. Ensemble pour le sang pour un appareil de traitement extracorporel du sang, l'ensemble pour le sang comprenant :
une unité de traitement du sang (2) ;
un circuit sanguin extracorporel accouplé à l'unité de traitement du sang (2) et comprenant une ligne de prélèvement de sang (6) et une ligne de retour de sang (7) pouvant être raccordées à un accès vasculaire d'un patient (P), éventuellement chacune parmi la ligne de prélèvement de sang (6) et la ligne de retour de sang (7) ayant un élément de raccordement d'extrémité respectif conçu pour être raccordé à l'accès vasculaire du patient (P) ;
un dispositif de déviation de fluide (21) selon l'une des revendications 1 à 14 précédentes, le dispositif de déviation de fluide (21) étant placé ou étant conçu pour être placé en ligne entre l'ensemble pour le sang de l'appareil (1) de traitement extracorporel du sang et l'accès vasculaire du patient (P), en particulier le conduit de prélèvement (23) et le conduit de retour (24) ayant un contre-élément de raccordement d'extrémité respectif conçu pour être accouplé ou accouplé à l'élément de raccordement d'extrémité de la ligne de prélèvement de sang (6) et de la ligne de retour de sang (7).
